# EUROPEAN PATENT SPECIFICATION

(11) **EP 3 496 639 B1**
(45) Date of publication and mention of the grant of the patent: **20.04.2022**
(21) Application number: 16784471.1
(22) Date of filing: 17.10.2016
(51) Int. Cl.: A61B 18/00

(54) **IMPROVED ENERGY-DELIVERING SYSTEM**
VERBESSERTES ENERGIEVERSORGUNGSSYSTEM
SYSTÈME AMÉLIORÉ DE DISTRIBUTION D'ÉNERGIE

(30) Priority: 12.08.2016 EP 16184127; 12.08.2016 EP 16184126
(43) Date of publication of application: 19.06.2019
(73) Proprietor: Medical Development Technologies S.A., 5823 Fentange (LU)
(72) Inventor: VAN LANGENHOVE, Glenn, 9820 Merelbeke (BE)
(74) Representative: Brantsandpatents bvba
(86) International application number: PCT/EP2016/074872
(87) International publication number: WO 2018/028804

(56) References cited:
- WO-A1-2013/149683
- US-A- 5 569 184
- US-A1- 2005 101 946
- US-A1- 2013 245 621
- US-A1- 2016 128 767

## Description

### TECHNICAL FIELD

The present invention concerns an improved energy-delivering system for providing energy to an implant device in an essentially wireless manner. More in particular, the present invention concerns an implant device comprising a pick-up coil and an ablation region and a catheter comprising an emitter coil adapted for producing time-varying magnetic fields at a position in a patient's body near the implant device, and preferably surrounded by the implant device.

### BACKGROUND

Implant devices such as stents are typically placed in vessels, e.g. arteries or veins, to keep the vessel open.

Implant devices can also be used to provide local treatment, e.g. to treat the surrounding tissue. An example of a system and implant device for local treatment is disclosed in document WO 2012/131107. Herein a system is disclosed comprising an implant device and external energy-providing means. The system is particularly developed for ablation of a vessel's wall from the inside, more specifically the system comprises implant devices which can be placed in one or more vessels, preferably in the pulmonary veins for the ablation of the wall of one or more pulmonary veins (PV) from the inside, preferably transmural ablation and preferably at the level of the antrum. Hereby, one or more implant devices can be implanted in the vessels and can subsequently be heated by external energy-providing means. The main objective of the ablation is to cause pulmonary vein isolation (PVI) by creating a lesion. PVI can solve the problem of atrial fibrillation (AF) by electrically isolating the one or more PVs from the heart tissue such that signals do not travel along myocardial sleeves along the PVs. The document thereto discloses a self-expanding implant device, adapted to be implanted and deployed within said vessel; whereby said implant comprises an ablation region along at least a portion of its length, said ablation region being adapted for surface contact with said vessel and said ablation region subtending at least a substantially complete circumferential band and being effective to ablate a signal-blocking path within said vessel upon application of energy to the implant.

US 2005/101946 A1 discloses a resonant circuit incorporated in a stent, which implantable in a pulmonary vein using known cardiac catheterization techniques. When an external RF field is generated at the resonant frequency of the stent, RF energy is re-radiated by the stent toward electroconductive tissue in the wall of the pulmonary vein, and produces a circumferential conduction block. The stent can be made of biodegradable materials, so that it eventually is resorbed. Following an ablation procedure, the stent may be left in situ. Repeated ablation can be performed using the inserted stent until it has been determined that the desired lesions have been formed. Furthermore, the same stent can potentially be used even years after being inserted should the treated arrhythmia reoccur or a new arrhythmia develop, thereby possibly obviating the need for an invasive procedure at that future time.

By developing an implant device which can be used for PVI, e.g. according to a system and methods disclosed in WO 2012/131107, the present inventors have found that use of a coil external to the patient's body to couple sufficient energy into the implant device may not be possible. Part of the emitted energy is lost by absorption in body tissue. It is in addition difficult to reach near perfect alignment of the external coil with respect to the implant device, which further increases the loss of emitted energy by absorption in body tissue. The emitted energy which is coupled into the pick-up coil of the implant device is converted by Joule heating into heat to ablate the vessel's wall. Part of the heat is drained by forced convection due to the blood flow. Increasing the emitted energy in order to obtain a sufficiently high temperature at the ablation region is not possible, because the present inventors have observed that such an increase causes skin burns on an animal's (pig) skin surface due to Eddy currents.

The device and system of the present invention overcome these problems.

### SUMMARY OF THE INVENTION

The present invention pertains to a system for wireless ablation of a pulmonary vein according to claim 1. Further advantageous embodiments are defined in the independent claims.

In an embodiment of the present invention, the catheter comprises a guiding tip at or near the distal end of the longitudinal shaft.

In another embodiment of the present invention, the catheter shaft has a guidewire lumen suitable for sliding the catheter over a guidewire.

In a preferred embodiment of the present invention, the pick-up coil of the implant device comprises the ablation region fully or partially.

In an embodiment of the present invention, the emitter coil comprises a fluxenhancing material.

In an embodiment of the present invention, the emitter coil comprises a central axis substantially parallel to the longitudinal axis of the catheter.

The guidewire or guiding tip can be used to bring the emitter coil of the catheter at a position in a patient's body near the implant device, and preferably surrounded by the implant device, thereby limiting the amount of body tissue in between the emitter and pick-up coils. The pick-up coil of the implant device comprises a central axis. The longitudinal axis of the catheter can be positioned substantially parallel to the central axis of the pick-up coil. When the central axis of the emitter coil is substantially parallel to the longitudinal axis of the catheter, it is also substantially parallel to the central axis of the pick-up coil, which results in a good coupling of the emitter and pick-up coils to transfer time-varying magnetic fields. The amount of emitted electromagnetic radiation lost by absorption in body tissue is hence significantly limited compared to the prior art.

In an embodiment, the time-varying magnetic field is a radiofrequent field, preferably comprising a frequency of between 10 kHz and 20 MHz, including e.g. 13.56 MHz, more preferably between 100 kHz and 1 MHz, such as 100 kHz, 200 kHz, 300 kHz, 400 kHz, 500 kHz, 600 kHz, 700 kHz, 800 kHz, 900 kHz, 1 Mhz, or any value therebetween.

In an embodiment of the present invention, the balloons are adapted to position upon inflation the catheter shaft at or near the center of the vein and/or the implant device, preferably by a substantial cylindrical symmetry of the outermost exterior regions of the inflated balloons with respect to the longitudinal axis of the catheter.

The central axes of the emitter and pick-up coils will reach upon inflation of the balloons near perfect coaxial alignment, when the emitter coil of the catheter is at a position in a patient's body near the implant device, and preferably surrounded by the implant device. The near perfect coaxial alignment of the emitter and pick-up coils ensures an even better transfer of time-varying magnetic fields from the emitter coil to the pick-up coil.

In addition, inflation of the balloons adapted for complete circumferential surface contact with the pulmonary vein and/or the implant device can stop the blood flow entirely, thereby stopping the drain of heat from the ablation region by forced convection due to the blood flow.

In an embodiment of the present invention, one of the balloons is adapted to thermally insulate the ablation region from blood in the vein upon inflation of the balloon.

In an embodiment of the present invention, at least one of the balloons is a perfusion balloon.

In an embodiment of the present invention, one or more balloons are coated with an anticoagulant, preferably heparin sulphate.

By thermally insulating the ablation region from the blood flow with an inflated balloon, the drain of heat from the ablation region by forced and/or natural convection is removed. With a perfusion balloon, limited blood flow can still be sustained, thereby significantly diminishing the chance of blood clots. Coating the balloons with an anticoagulant can also significantly diminish the chance of blood clots.

In an embodiment of the present invention, the longitudinal body of the catheter comprises an additional transfer lumen with a distal end and a proximal end, and the distal end of the transfer lumen is in communication with the exterior region of the balloons via one or more openings in the catheter shaft at or near the distal end of the catheter, and the proximal end of the transfer lumen is adapted for connection with a fluid delivery and/or drainage system.

In an embodiment of the present invention, the implant device comprises a central axis, and the implant device is radially expandable in directions perpendicular to its central axis, and at least one of the balloons is uninflated and designed for support of the unexpanded implant device, and at least one of the balloons designed for support of the implant device is able to withstand pressures sufficient to radially expand the implant device. The catheter may comprise a movable protector sheath around the catheter shaft suitable for protection of the unexpanded implant device supported by an uninflated balloon.

The implant device of the present invention can also be inserted in the vein, positioned, radially expanded by inflation of a supporting balloon, mounted in the wall of the vein, and its ablation region heated, with a single insertion of a single catheter in the patient's body. The supporting balloon may also be used to thermally insulate the blood in the vein from the ablation region and/or to stop the blood flow in the vein.

### DESCRIPTION OF THE FIGURES

**Figure 1** schematically represents an embodiment of the present invention comprising an implant device, a catheter, and an electric power source.
**Figure 2** schematically represents an embodiment of a catheter according to the present invention, comprising a guiding tip near its distal end.
**Figure 3** schematically represents an embodiment of a guidewire and a catheter according to the present invention, the catheter comprising a guiding lumen suitable for sliding the catheter over the guidewire.
**Figures 4****,** **5, and 6** schematically represent embodiments of an implant device according to the present invention.
**Figures 7, 8, and 9** schematically represent embodiments of a portion of the distal end of a catheter according to the present invention, comprising an emitter coil and wiring.
**Figures 10, 11****,** **14, and 15** schematically represent embodiments of the distal end of a catheter according to the present invention, comprising an emitter coil, wiring, a balloon, and an inflation lumen.
**Figures 12** **and** **17** schematically represent embodiments of an implant device according to the present invention and embodiments of the distal end of a catheter according to the present invention.
**Figures 13** **and** **16** schematically represent a vessel, embodiments of an implant device according to the present invention, and embodiments of the distal end of a catheter according to the present invention.
**Figure 18** schematically represents an embodiment of the distal end of a catheter according to the present invention comprising a portion of the catheter shaft, an emitter coil, wiring, an uninflated balloon, an inflation lumen, an implant device and a movable protector sheath.

### DETAILED DESCRIPTION

The present invention pertains to a system for wireless ablation of a pulmonary vein comprising
a. an implant device provided with a pick-up coil and an ablation region, whereby the ablation region of the implant device is adapted for surface contact with the pulmonary vein and for subtending at least a substantially complete circumferential band for ablating a signal-blocking path within the pulmonary vein upon heating of the ablation region,
b. a catheter comprising a longitudinal shaft with a distal end, a proximal end, and a longitudinal body in between, whereby the catheter comprises a longitudinal axis along the longitudinal shaft, and whereby the catheter further comprises an emitter coil at or near the distal end, and whereby the longitudinal body of the catheter further comprises a wiring lumen comprising electrical wiring extending from the distal end to the proximal end, and whereby the electrical wiring is connected at or near the distal end with the emitter coil,
c. an electric power source which can be connected to the wiring via the proximal end of the catheter shaft for the generation of a time-varying magnetic field with the emitter coil;.
and in which:
a. the catheter shaft comprises one or more balloons at or near its distal end, each balloon comprising one or more air-tight connections to the catheter shaft, each balloon further comprising an interior and an exterior region, the exterior regions of the balloons adapted for complete circumferential surface contact with the vein and/or the implant device upon inflation;
b. the longitudinal body of the catheter shaft comprises an inflation lumen, the inflation lumen comprising a distal end and a proximal end, the proximal end of the inflation lumen adapted for connection with a pressurizing means; and
c. the interior regions of the balloons are in communication with the distal end of the inflation lumen;
in which the longitudinal body of the catheter comprises an additional transfer lumen with a distal end and a proximal end, and in which the distal end of the transfer lumen is in communication with the exterior region of the balloons via one or more openings in the catheter shaft at or near the distal end of the catheter, and in which the proximal end of the transfer lumen is adapted for connection with a fluid delivery and/or drainage system.

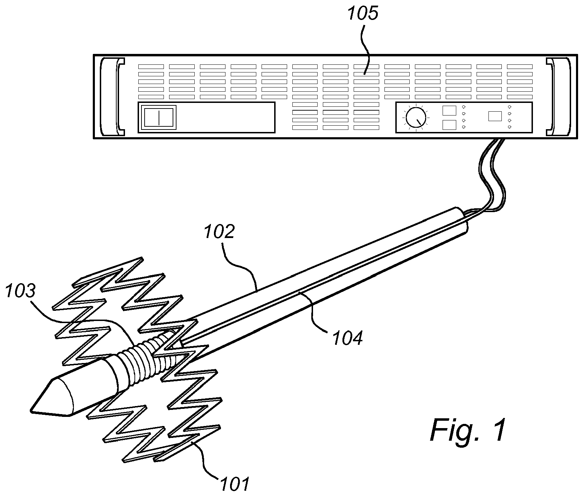

**FIG. 1** schematically represents an embodiment of the present invention comprising a radially expandable implant device **101,** a catheter **102,** an emitter coil **103** at the distal end, electrical wiring **104** extending from the distal end to the proximal end, connected at the distal end with the emitter coil **103** and at the proximal end with an electric power source **105.**

In an embodiment of the present invention, the catheter comprises a guiding tip at or near the distal end of the longitudinal shaft.

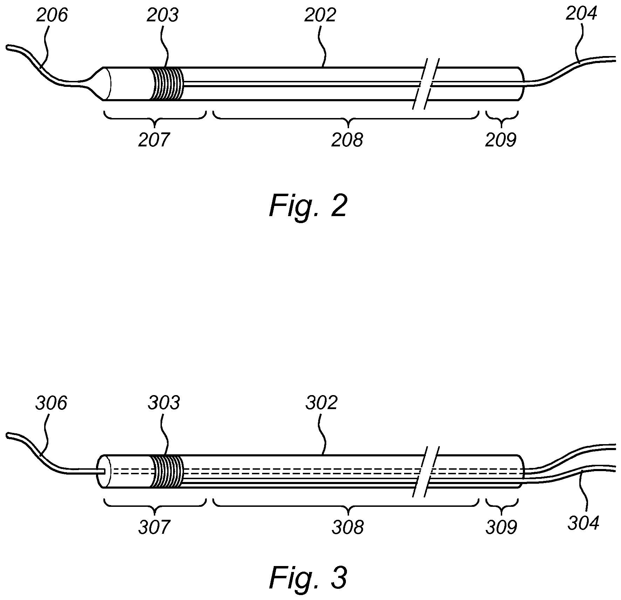

**FIG. 2** schematically represents an embodiment of a catheter according to the present invention, comprising a longitudinal shaft **202** with a distal end **207,** a proximal end **209,** and a longitudinal body **208** in between, the distal end **207** of the catheter shaft comprising an emitter coil **203** and a guiding tip **206.** The emitter coil **203** is connected to electrical wiring **204** extending from the distal end to the proximal end.

In another embodiment of the present invention, the catheter shaft has a guiding lumen suitable for sliding the catheter over a guidewire.

**FIG. 3** schematically represents a guidewire **306** and an embodiment of a catheter according to the present invention, comprising a longitudinal shaft **302** with a distal end **307,** a proximal end **309,** and a longitudinal body **308** in between, the distal end **307** of the catheter shaft comprising an emitter coil **303.** The emitter coil **303** is connected to electrical wiring **304** extending from the distal end to the proximal end. The catheter shaft has a guiding lumen suitable for sliding the catheter over the guidewire **306.**

In a preferred embodiment of the present invention, the pick-up coil of the implant device comprises the ablation region fully or partially.

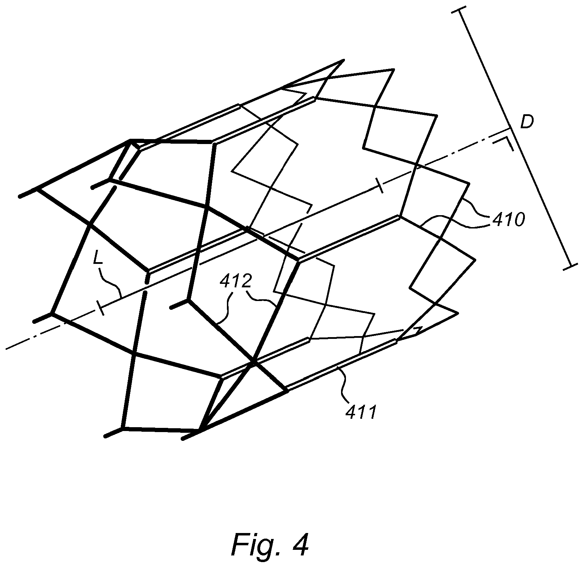

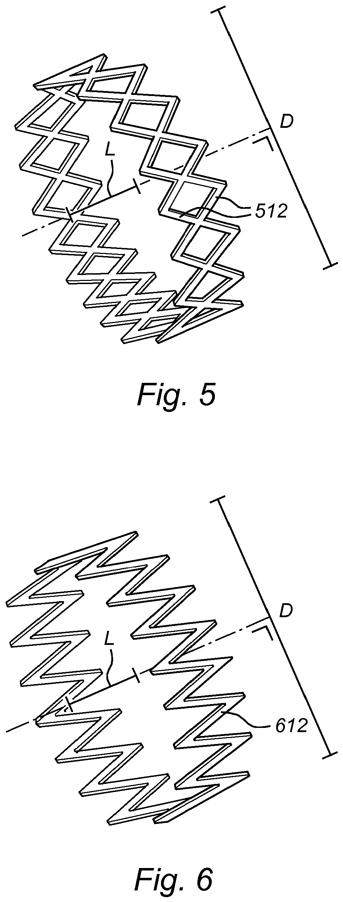

**FIGS. 4****,** **5, and 6** schematically represent embodiments of an implant device according to the present invention. The implant devices comprise a central axis **L,** and are radially expandable in directions perpendicular to the central axis. The pickup coils of the implant devices also comprise the ablation regions **412, 512, 612** fully. The implant device in **FIG. 4** comprises in addition to the radially expandable ablation region **412** also a second radially expandable ring of diamond-shaped elements **410** which are connected to the ablation region by struts **411.**

In an embodiment of the present invention, the emitter coil comprises a fluxenhancing material.

In a preferred embodiment of the present invention, the emitter coil comprises a central axis substantially parallel to the longitudinal axis of the catheter.

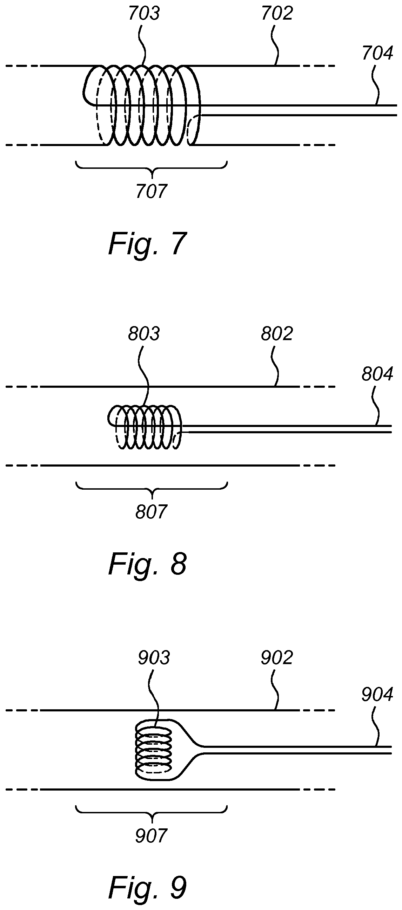

**FIGS. 7, 8, and 9** schematically represent embodiments of a portion of the distal end **707, 807, 907** of the catheter shaft **702, 802, 902** according to the present invention, comprising an emitter coil **703, 803, 903** connected to electrical wiring **704, 804, 904** extending from the distal end to the proximal end of the catheter shaft. In **FIGS. 7 and 8** the central axis of the emitter coil **703, 803** is substantially parallel to the longitudinal axis of the catheter. In **FIG. 9** the central axis of the emitter coil **903** is not substantially parallel to the longitudinal axis of the catheter.

The guidewire or guiding tip can be used to bring the emitter coil of the catheter at a position in a patient's body near the implant device, and preferably surrounded by the implant device, thereby limiting the amount of body tissue in between the emitter and pick-up coils. The pick-up coil of the implant device comprises a central axis. The longitudinal axis of the catheter can be positioned substantially parallel to the central axis of the pick-up coil. When the central axis of the emitter coil is substantially parallel to the longitudinal axis of the catheter, it is also substantially parallel to the central axis of the pick-up coil, which results in a good coupling of the emitter and pick-up coils to transfer time-varying magnetic fields. The amount of emitted electromagnetic radiation lost by absorption in body tissue is hence significantly limited compared to the prior art.

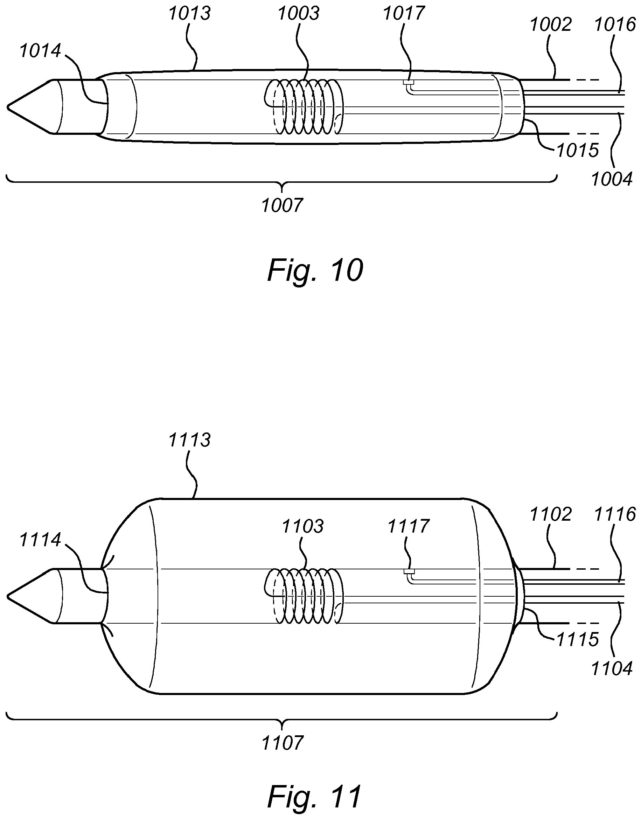

**FIGS. 10 and 11** schematically represent embodiments of the distal end **1007, 1107** of a catheter shaft **1002, 1102** according to the present invention comprising a balloon **1013, 1113,** whereby the balloon comprises two air-tight connections **1014, 1114** and **1015, 1115** to the catheter shaft. The distal end of an inflation lumen **1016, 1116** is in communication with the interior of the balloon **1013, 1113** by means of an opening **1017, 1117** in the catheter shaft. In **FIG. 10** the balloon is not inflated. In **FIG. 11** the balloon is inflated.

In a preferred embodiment of the present invention, the balloons are adapted to position upon inflation the catheter shaft at or near the center of the vein and/or the implant device, preferably by a substantial cylindrical symmetry of the outermost exterior regions of the inflated balloons with respect to the longitudinal axis of the catheter.

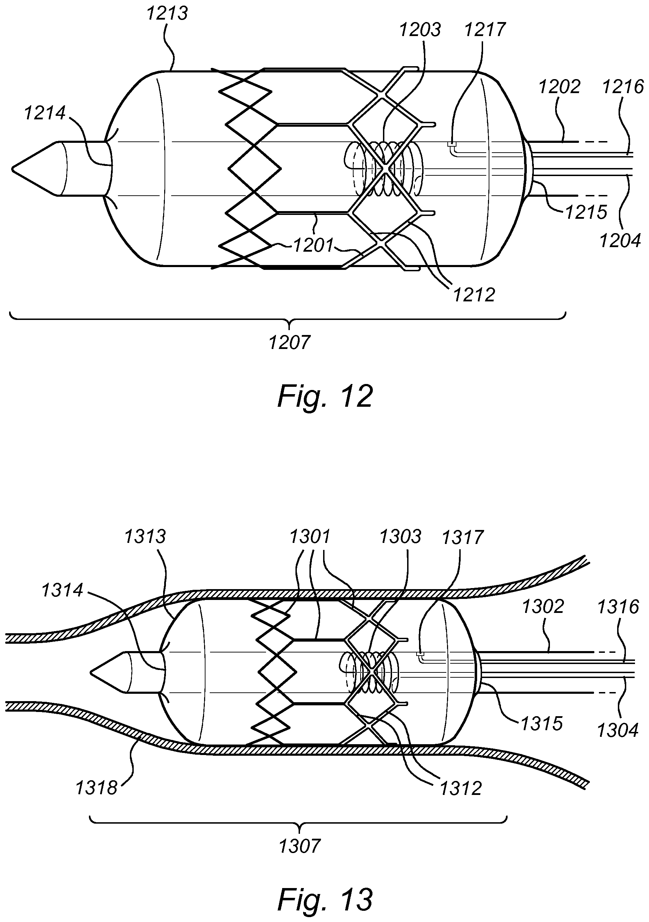

**FIGS. 12 and 13** schematically represent embodiments of an implant device **1201, 1301** according to the present invention and embodiments of the distal end **1207, 1307** of a catheter shaft **1202, 1302** according to the present invention comprising a balloon **1213, 1313,** whereby the balloon comprises two air-tight connections **1214, 1314** and **1215, 1315** to the catheter shaft. The distal end of an inflation lumen **1216, 1316** is in communication with the interior of the balloon **1213, 1313** by means of an opening **1217, 1317** in the catheter shaft. The outermost exterior region of the inflated balloon **1213, 1313** has cylindrical symmetry with respect to the longitudinal axis of the catheter, thereby aligning central axis of the emitter coil **1203, 1303** coaxially with the central axis of the pick-up coil of the implant device **1201, 1301.**

The central axes of the emitter and pick-up coils will reach upon inflation of the balloons near perfect coaxial alignment, when the emitter coil of the catheter is at a position in a patient's body near the implant device, and preferably surrounded by the implant device. The near perfect coaxial alignment of the emitter and pick-up coils ensures an even better transfer of time-varying magnetic fields from the emitter coil to the pick-up coil.

In addition, inflation of the balloons adapted for complete circumferential surface contact with the pulmonary vein and/or the implant device can stop the blood flow entirely, thereby stopping the drain of heat from the ablation region by forced convection due to the blood flow.

In an embodiment of the present invention, one of the balloons is adapted to thermally insulate the ablation region from blood in the vein upon inflation of the balloon.

In **FIG. 13** the inflated balloon **1313** is in complete circumferential surface contact with the implant device **1301** as well as the vein **1318.** The ablation region **1312** is thereby thermally insulated from blood in the vein.

In an embodiment of the present invention, at least one of the balloons is a perfusion balloon.

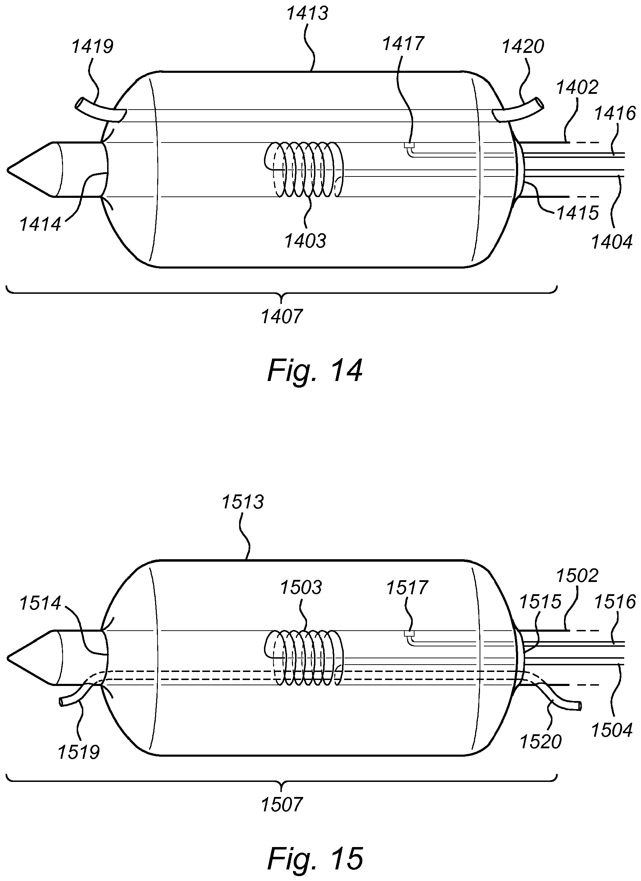

**FIGS. 14 and 15** schematically represent embodiments of the distal end **1407, 1507** of a catheter shaft **1402, 1502** according to the present invention comprising a balloon **1413, 1513,** whereby the balloon comprises two air-tight connections **1414, 1514** and **1415, 1515** to the catheter shaft. The balloon **1419** in **FIG. 14** comprises a perfusion channel with two ends **1419** and **1420,** which can allow limited blood flow in a vein even if the outermost exterior region of the inflated balloon is in complete circumferential surface contact with the implant device and/or the vein to insulate the ablation region from the blood. The perfusion channel in the balloon **1413** in **FIG. 14** comprises a tube. The perfusion channel in the balloon can also comprise a lumen comprising a wall of essentially the same material and thickness as the outermost wall of the balloon. A perfusion balloon is a term denoting a balloon comprising a perfusion channel. **FIG. 15** schematically represents an embodiment of the present invention, in which the catheter shaft comprises a perfusion channel with two ends **1519** and **1520.**

In an embodiment of the present invention, one or more balloons are coated with an anticoagulant, preferably heparin sulphate.

By thermally insulating the ablation region from the blood flow with an inflated balloon, the drain of heat from the ablation region by forced and/or natural convection is removed. With a perfusion balloon, limited blood flow can still be sustained, thereby significantly diminishing the chance of blood clots. Coating the balloons with an anticoagulant can also significantly diminish the chance of blood clots.

In an embodiment of the present invention, the longitudinal body of the catheter comprises an additional transfer lumen with a distal end and a proximal end, and the distal end of the transfer lumen is in communication with the exterior region of the balloons via one or more openings in the catheter shaft at or near the distal end of the catheter, and the proximal end of the transfer lumen is adapted for connection with a fluid delivery and/or drainage system.

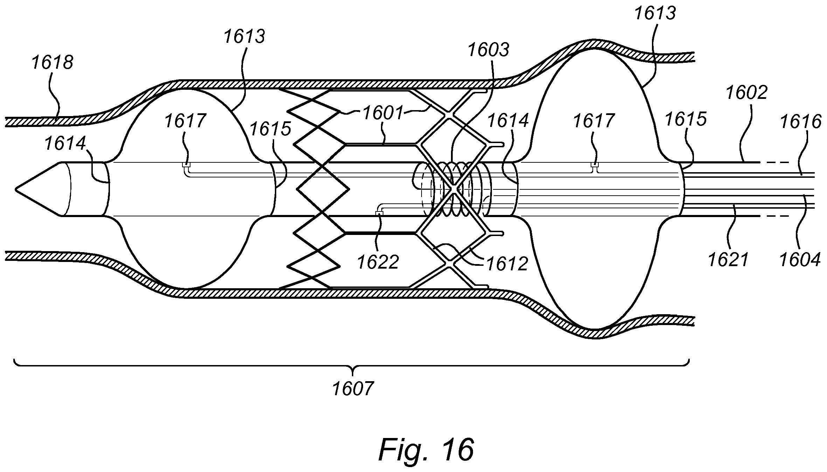

**FIG. 16** schematically represents a vessel **1618,** an embodiment of an implant device **1601** according to the present invention, and an embodiment of the distal end **1607** of a catheter shaft **1602** according to the present invention comprising two balloons **1613** with cylindrical symmetry with respect to the longitudinal axis of the catheter and adapted for complete circumferential surface contact with the vessel **1618.** The catheter shaft **1602** further comprises an emitter coil **1603** located in between the two balloons **1613.** The catheter shaft **1602** further comprises a transfer lumen **1621,** which is in communication with the exterior region of the balloons **1613** by means of an opening **1622** in the catheter shaft in between the two balloons. The emitter coil **1603** is positioned near the implant device **1601,** preferably surrounded by the implant device **1601.** Inflation of the balloons **1613** can align the central axes of the emitter coil and the pick-up coil coaxially and can stop the blood flow in the vessel. For certain vessels and/or regions in vessels, the two balloons can have a different shape and or size in order to reach complete circumferential surface contact. Blood in the region spanned by the vessel **1618** and the exterior regions of the two inflated balloons **1613** can be replaced with another harmless fluid by means of the transfer lumen **1621,** thereby reducing the chance of blood clots due to heat from the ablation region.

In an embodiment of the present invention, the implant device comprises a central axis, and the implant device is radially expandable in directions perpendicular to its central axis, and at least one of the balloons is uninflated and designed for support of the unexpanded implant device, and at least one of the balloons designed for support of the implant device is able to withstand pressures sufficient to radially expand the implant device. The catheter may comprise a movable protector sheath around the catheter shaft suitable for protection of the unexpanded implant device supported by an uninflated balloon.

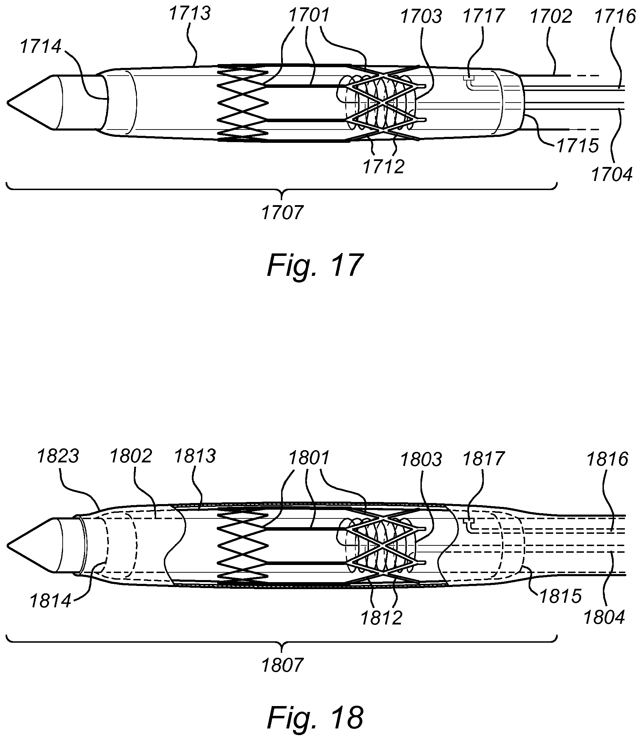

**FIG. 17 and 18** schematically represent the distal end **1707, 1807** of a catheter shaft **1702, 1802** according to the present invention comprising an emitter coil **1703, 1803,** electrical wiring **1704, 1804,** an uninflated balloon **1713, 1813,** an inflation lumen **1716, 1816,** a connection **1717, 1817** to bring the inflation lumen **1716, 1816** in communication with the interior of the balloon **1713, 1813,** and an unexpanded implant device **1701, 1801** supported by the uninflated balloon **1713, 1813.** In **FIG. 18****,** the catheter further comprises a movable protector sheath **1823** around the catheter shaft **1802,** uninflated balloon **1813,** and implant device **1801.**

The implant device of the present invention can also be inserted in the vein, positioned, radially expanded by inflation of a supporting balloon, mounted in the wall of the vein, and its ablation region heated, with a single insertion of a single catheter in the patient's body. The supporting balloon may also be used to thermally insulate the blood in the vein from the ablation region and/or to stop the blood flow in the vein.

In an embodiment of the present invention, the implant device comprises a thermoactive coating with an activation temperature above a predefined temperature, preferably above 37°C, more preferably above 40°C, such as 41°C, 42°C, 43°C, 44°C, 45°C, to activate the thermoactive coating simultaneous to and/or after heating of the ablation region.

In an embodiment of the present invention, the implant device comprises substances which are released from the implant device upon or after activation of the thermoactive coating.

In an embodiment of the present invention, the emitter coil and/or pick-up coil comprise a biocompatible metal, preferably nitinol.

### EXAMPLE

The example pertains to an embodiment of the present invention for wireless ablation of a pulmonary vein as illustrated in **FIG. 18****,** in which the distal end **1807** of the catheter shaft **1802** comprises
a. an emitter coil **1813** comprising a central axis substantially parallel to the longitudinal axis of the catheter and connected to electrical wiring **1804** extending from the distal end **1807** to the proximal end, whereby the electrical wiring **1804** can be connected at or near the proximal end of the catheter shaft to an electric power source,
b. an uninflated balloon **1813** with two air-tight connections **1814** and **1815** to the catheter shaft **1802,** the balloon comprising an anticoagulant coating comprising heparin sulphate,
c. an inflation lumen **1816** in communication with the interior of the balloon **1813** by means of an opening **1817** in the catheter shaft **1802** and which can be connected at or near the proximal end to a pressurizing means,
d. an implant device **1801,** radially expandable in directions perpendicular to its central axis and supported by the uninflated balloon, whereby the ablation region **1812** of the implant device surrounds the emitter coil **1804,** and whereby the implant device comprises a thermoactive coating with an activation temperature preferably above 37°C, more preferably above 40°C, such as 41°C, 42°C, 43°C, 44°C, 45°C,
e. a movable protector sheath **1823,** covering the catheter shaft **1802,** the uninflated balloon **1813,** and the unexpanded implant device **1801.**

The balloon **1813** and the air-tight connections **1814, 1815** are further adapted
a. to withstand pressures sufficient to radially expand the implant device **1801,**
b. to yield upon inflation an outermost exterior region of the balloon **1813** with substantial cylindrical symmetry,
c. for complete circumferential surface contact with the pulmonary vein,
d. to withstand heating of the ablation region **1812,** and
e. to thermally insulate the ablation region from blood in the pulmonary vein.

A guidewire or guiding tip can be used to bring the distal end **1807** of the catheter shaft **1802** in the pulmonary vein of the patient, whereby the ablation region **1812** of the unexpanded implant device **1801** is positioned at a location where the pulmonary vein needs to be insulated. The movable protector sheath **1823** ensures that during insertion of the catheter in the patient's body, the implant device **1801** cannot come into contact with any veins. Once the catheter is positioned as desired, the movable protector sheath **1823** is retracted in the direction of the proximal end of the catheter shaft, thereby exposing the uninflated balloon **1813** and unexpanded implant device **1801.** The interior of the balloon **1813** is in communication with the inflation lumen **1816** by means of an opening **1817** in the catheter shaft **1802.** With a pressurizing means connected to the proximal end of the inflation lumen **1816,** the balloon **1813** can be inflated, thereby radially expanding the implant device **1801** as well.

As illustrated in **FIG. 13****,** inflation of the balloon **1313**
a. mounts the implant device **1301** in the vein **1318,**
b. stops blood flow in the vein **1318,**
c. thermally insulates blood in the vein **1318** from the ablation region **1312** of the implant device **1301,**
d. ensures substantial parallel alignment of the central axes of the emitter coil **1303** and the pick-up coil of the implant device **1301,**
e. even ensures near perfect coaxial alignment of the central axes of the emitter coil **1303** and the pick-up coil of the implant device **1301,**
f. positions the emitter coil **1303** inside the ablation region **1312** of the implant device **1301.**

The chance of blood clot formation in the vein **1318** is limited due to the anticoagulant coating of the balloon **1313** comprising heparin sulphate. The emitter coil **1303** can be connected to the electric power source by means of electrical wiring **1304** extending from the distal end to the proximal end of the catheter shaft **1302.** With an alternating current of the electric power source, the emitter coil **1303** emits a time-varying magnetic field, which is optimally coupled to the pickup coil of the implant device **1301** due to the absence of body tissue in between the emitter and pick-up coils and due to the near perfect coaxial alignment of the central axes of the emitter and pick-up coils. The time-varying magnetic field induces a current in the pick-up coil, which is converted in the ablation region **1312** into heat by Joule heating. As blood cannot reach the ablation region **1312** due to the inflated balloon **1313,** the heat cannot be drained by natural or forced convection. In order to limit the chance of blood clot formation even more, the balloon **1313** can be a perfusion balloon, or the catheter can comprise a perfusion lumen. When the ablation region reaches a sufficiently high temperature, the thermoactive coating of the implant device is released, to produce a lesion of limited necrosis and/or neurotoxicity during the ablation.

## Claims

1. System for wireless ablation of a pulmonary vein comprising
a. an implant device (101, 1201, 1301) provided with a pick-up coil and an ablation region, (412,512,612) whereby the ablation region of the implant device is adapted for surface contact with the pulmonary vein and for subtending at least a substantially complete circumferential band for ablating a signal-blocking path within the pulmonary vein upon heating of the ablation region,
b. a catheter (102) comprising a longitudinal shaft with a distal end, a proximal end, and a longitudinal body in between, whereby the catheter comprises a longitudinal axis along the longitudinal shaft, and whereby the catheter further comprises an emitter coil (103, 203, 303) at or near the distal end, and whereby the longitudinal body of the catheter further comprises a wiring lumen comprising electrical wiring extending from the distal end to the proximal end, and whereby the electrical wiring is connected at or near the distal end with the emitter coil, and
c. an electric power source (105) which can be connected to the wiring via the proximal end of the catheter shaft for the generation of a time- varying magnetic field with the emitter coil;
and in which
a. the catheter shaft (1002, 1102, 1202, 1302) comprises one or more balloons (1013, 1113, 1213, 1313) at or near its distal end, each balloon comprising one or more air-tight connections (1214, 1314, 1215, 1315) to the catheter shaft, each balloon further comprising an interior and an exterior region, the exterior regions of the balloons adapted for complete circumferential surface contact with the vein and/or the implant device upon inflation,
b. the longitudinal body of the catheter shaft comprises an inflation lumen, (1016, 1116, 1216, 1316) the inflation lumen comprising a distal end and a proximal end, the proximal end of the inflation lumen adapted for connection with a pressurizing means, and
c. the interior regions of the balloons are in communication with the distal end of the inflation lumen; in which the longitudinal body of the catheter comprises an additional transfer lumen (1621) with a distal end and a proximal end, and in which the distal end of the transfer lumen is in communication with the exterior region of the balloons via one or more openings in the catheter shaft at or near the distal end of the catheter, and in which the proximal end of the transfer lumen is adapted for connection with a fluid delivery and/or drainage system.

2. System according to claim 1, in which the catheter comprises a guiding tip at or near the distal end of the longitudinal shaft, or the catheter shaft has a guidewire lumen suitable for sliding the catheter over a guidewire.

3. System according to any one of the previous claims, in which the pick-up coil of the implant device fully or partially comprises the ablation region.

4. System according to any one of the previous claims, in which the emitter coil comprises a flux-concentrating material.

5. System according to any one of the previous claims, in which the emitter coil comprises a central axis substantially parallel to the longitudinal axis of the catheter.

6. System according to claim 5, in which the balloons are adapted to position upon inflation the catheter shaft at or near the center of the vein and/or the implant device, preferably by a substantial cylindrical symmetry of the outermost exterior regions of the inflated balloons with respect to the longitudinal axis of the catheter.

7. System according to any one of claims 1 to 6, in which at least one of the balloons is adapted to thermally insulate the ablation region from blood in the vein upon inflation of the balloon.

8. System according to any one of claims 1 to 7, in which the implant device comprises a central axis, and in which the implant device is radially expandable in directions perpendicular to its central axis, and in which at least one of the balloons is uninflated and designed for support of the unexpanded implant device, and in which at least one of the balloons designed for support of the implant device is able to withstand pressures sufficient to radially expand the implant device.

9. System according to claim 8, in which the catheter comprises a movable protector sheath around the catheter shaft suitable for protection of the unexpanded implant device supported by an uninflated balloon.

10. System according to any one of claims 1 to 9, in which at least one of the balloons is a perfusion balloon, or the catheter shaft comprises a perfusion channel.

11. System according to any one of claims 1 to 10, in which at least one of the balloons is coated with an anticoagulant, preferably heparin sulphate.

12. System according to any one of the previous claims, in which the implant device comprises a thermoactive coating with an activation temperature above a predefined temperature, preferably above 37°C, more preferably above 40°C, such as 41°C, 42°C, 43°C, 44°C, 45°C, to activate the thermoactive coating simultaneous to and/or after heating of the ablation region, wherein preferably the implant device comprises substances which are released from the implant device upon or after activation of the thermoactive coating.

13. System according to any one of the previous claims, wherein the implant device comprises a central axis (L), wherein the implant device is radially expandable in directions perpendicular to the central axis, wherein the pick-up coil comprises the ablation region (412), wherein the ablation region is radially expandable, and wherein the implant device comprises a second radially expandable ring of diamond-shaped elements (410) which are connected to the ablation region by struts (411).

## Patentansprüche

1. System zur drahtlosen Ablation einer Pulmonarvene, Folgendes umfassend:
a. eine Implantatvorrichtung (101, 1201, 1301), die mit einer Pickup-Spule und einem Ablationsbereich (412, 512, 612) versehen ist, wobei der Ablationsbereich der Implantatvorrichtung für den Oberflächenkontakt mit der Pulmonarvene und zum Entgegensetzen mindestens eines im Wesentlichen vollständigen umlaufenden Bandes zum Ablatieren eines Signalblockierungsweges innerhalb der Pulmonarvene auf das Erwärmen des Ablationsbereichs hin eingerichtet ist,
b. einen Katheter (102), der einen sich längs erstreckenden Schaft mit einem distalen Ende, einem proximalen Ende und einem sich längs erstreckenden Hauptteil dazwischen umfasst, wobei der Katheter entlang des sich längs erstreckenden Schafts eine Längsachse umfasst und wobei der Katheter am oder nahe dem distalen Ende ferner eine Emitterspule (103, 203, 303) umfasst und wobei der sich längs erstreckende Hauptteil des Katheters ferner ein Verkabelungslumen umfasst, das elektrische Verkabelung umfasst, die sich von dem distalen Ende zu dem proximalen Ende erstreckt, und wobei die elektrische Verkabelung an oder nahe dem distalen Ende mit der Emitterspule verbunden ist, und
c. eine Elektronenergiequelle (105), die mittels des proximalen Endes des Katheterschafts mit der Verkabelung verbunden werden kann, um mit der Emitterspule ein zeitlich variierendes Magnetfeld zu erzeugen, und wobei
a. der Katheterschaft (10002, 1102, 1202, 1302) nahe seinem distalen Ende einen oder mehrere Ballons (1013, 1113, 1213, 1313) umfasst, wobei jeder Ballon eine oder mehrere luftdichte Verbindungen (1214, 1314, 1215, 1315) mit dem Katheterschaft umfasst, wobei jeder Ballon ferner einen inneren und einen äußeren Bereich umfasst, wobei die äußeren Bereiche der Ballons dafür eingerichtet sind, auf das Aufblasen hin mit der umlaufenden Oberfläche vollständig mit der Vene und/oder der Implantatvorrichtung in Kontakt zu stehen,
b. der sich längs erstreckende Hauptteil des Katheterschafts ein Aufblaslumen (1016, 1116, 1216, 1316) umfasst, wobei das Aufblaslumen ein distales Ende und ein proximales Ende umfasst, wobei das proximale Ende des Aufblaslumens für die Verbindung mit einem Druckbaufschlagungsmittel eingerichtet ist, und
c. die inneren Bereiche der Ballons in Verbindung mit dem distalen Ende des Aufblaslumens stehen,
wobei der sich längs erstreckende Hauptteil des Katheters ein zusätzliches Transferlumen (1621) mit einem distalen Ende und einem proximalen Ende umfasst und wobei das distale Ende des Transferlumens über eine oder mehrere Öffnungen in dem Katheterschaft an oder nahe dem distalen Ende des Katheters in Verbindung mit dem äußeren Bereich der Ballons steht und wobei das proximale Ende des Transfervolumens für die Verbindung mit einem Fluidzufuhr- und/oder -ablaufsystem eingerichtet ist.

2. System nach Anspruch 1, wobei der Katheter an oder nahe dem distalen Ende des sich längs erstreckenden Schafts eine Führungsspitze umfasst oder der Katheterschaft ein Führungsdrahtlumen aufweist, das geeignet ist, den Katheter über einen Führungsdraht gleiten zu lassen.

3. System nach einem der vorhergehenden Ansprüche, wobei die Pickup-Spule der Implantatvorrichtung die Ablationsregion vollständig oder teilweise umfasst.

4. System nach einem der vorhergehenden Ansprüche, wobei die Emitterspule ein Fluxkonzentrationsmaterial umfasst.

5. System nach einem der vorhergehenden Ansprüche, wobei die Emitterspule eine Mittelachse umfasst, die im Wesentlichen parallel zur Längsachse des Katheters liegt.

6. System nach Anspruch 5, wobei die Ballons dafür eingerichtet sind, sich auf das Aufblasen des Katheterschafts hin an oder nahe der Mitte der Vene und/oder der Implantatvorrichtung zu positionieren, vorzugsweise durch eine wesentliche zylindrische Symmetrie der äußersten Außenbereiche der aufgeblasenen Ballons im Verhältnis zur Längsachse des Katheters.

7. System nach einem der Ansprüche 1 bis 6, wobei mindestens einer der Ballons dafür eingerichtet ist, auf das Aufblasen des Ballons hin den Ablationsbereich thermisch vom Blut in der Vene zu isolieren.

8. System nach einem der Ansprüche 1 bis 7, wobei die Implantatvorrichtung eine Mittelachse umfasst und wobei die Implantatvorrichtung radial ausfahrbar ist, in Richtungen, die senkrecht zu deren Mittelachse liegen, und wobei mindestens einer der Ballons nicht aufgeblasen und dafür gestaltet ist, die nicht ausgefahrene Implantatvorrichtung zu tragen, und wobei mindestens einer der Ballons, der zum Tragen der Implantatvorrichtung gestaltet ist, in der Lage ist, Drücken zu widerstehen, die ausreichen, um die Implantatvorrichtung radial auszufahren.

9. System nach Anspruch 8, wobei der Katheter rings um den Katheterschaft eine bewegliche Schutzhülle umfasst, die geeignet ist, die von einem nicht aufgeblasenen Ballon getragene, nicht ausgefahrene Implantatvorrichtung zu schützen.

10. System nach einem der Ansprüche 1 bis 9, wobei mindestens einer der Ballons ein Perfusionsballon ist oder der Katheterschaft einen Perfusionskanal umfasst.

11. System nach einem der Ansprüche 1 bis 10, wobei mindestens einer der Ballons mit einem Antikoagulans beschichtet ist, vorzugsweise Heparinsulfat.

12. System nach einem der vorhergehenden Ansprüche, wobei die Implantatvorrichtung eine thermoaktive Beschichtung mit einer Aktivierungstemperatur über einer vordefinierten Temperatur umfasst, vorzugsweise über 37 °C, bevorzugter über 40 °C, wie beispielsweise 41 °C, 42 °C, 43 °C, 44 °C, 45 °C, um die thermoaktive Beschichtung gleichzeitig und/oder nach einem Erwärmen des Ablationsbereichs zu aktivieren, wobei die Implantatvorrichtung vorzugsweise Substanzen umfasst, die auf die Aktivierung der thermoaktiven Beschichtung hin oder danach aus der Implantatvorrichtung abgegeben werden.

13. System nach einem der vorhergehenden Ansprüche, wobei die Implantatvorrichtung eine Mittelachse (L) umfasst, wobei die Implantatvorrichtung in Richtungen, die senkrecht zur Mittelachse liegen, radial ausfahrbar ist, wobei die Pickup-Spule den Ablationsbereich (412) umfasst, wobei der Ablationsbereich radial ausfahrbar ist und wobei die Implantatvorrichtung einen zweiten radial ausfahrbaren Ring aus rautenförmigen Elementen (410) umfasst, die durch Streben (411) mit dem Ablationsbereich verbunden sind.

## Revendications

1. Système pour l'ablation sans fil d'une veine pulmonaire comprenant
a. un dispositif d'implantation (101, 1201, 1301) pourvu d'une bobine de captage et d'une zone d'ablation, la zone d'ablation (412, 512, 612) étant conçue pour un contact de surface avec la veine pulmonaire et pour sous-tendre au moins une bande circonférentielle sensiblement complète pour l'ablation d'un trajet de blocage de signal dans la veine pulmonaire lors du chauffage de la zone d'ablation,
b. un cathéter (102) comprenant une tige longitudinale avec une extrémité distale, une extrémité proximale et un corps longitudinal entre les deux, moyennant quoi le cathéter comprend un axe longitudinal le long de la tige longitudinale, et moyennant quoi le cathéter comprend en outre une bobine émettrice (103, 203, 303) à ou près de l'extrémité distale, et moyennant quoi le corps longitudinal du cathéter comprend en outre une lumière de câblage comprenant un câblage électrique s'étendant de l'extrémité distale à l'extrémité proximale, et moyennant quoi le câblage électrique est connecté à ou près de l'extrémité distale avec la bobine émettrice, et
c. une source d'énergie électrique (105) qui peut être connectée au câblage via l'extrémité proximale de la tige du cathéter pour la génération d'un champ magnétique variant dans le temps avec la bobine émettrice; et dans lequel
a. la tige de cathéter (10002, 1102, 1202, 1302) comprend un ou plusieurs ballons à ou près de son extrémité distale, chaque ballon comprenant une ou plusieurs connexions étanches à l'air (1214, 1314, 1215, 1315) à la tige de cathéter, chaque ballon comprenant en outre une zone intérieure et une zone extérieure, les zones extérieures des ballons étant conçues pour un contact de surface circonférentiel complet avec la veine et/ou le dispositif d'implantation lors du gonflage,
b. le corps longitudinal de la tige du cathéter comprend une lumière de gonflage (1016, 1116, 1216, 1316), la lumière de gonflage comprenant une extrémité distale et une extrémité proximale, l'extrémité proximale de la lumière de gonflage étant conçue pour être connectée à un moyen de pressurisation, et
c. les zones intérieures des ballons sont en communication avec l'extrémité distale de la lumière de gonflage;
dans lequel le corps longitudinal du cathéter comprend une lumière de transfert supplémentaire (1621) avec une extrémité distale et une extrémité proximale, et dans lequel l'extrémité distale de la lumière de transfert est en communication avec la zone extérieure des ballons par l'intermédiaire d'une ou plusieurs ouvertures dans la tige du cathéter au niveau ou à proximité de l'extrémité distale du cathéter, et dans lequel l'extrémité proximale de la lumière de transfert est conçue pour se connecter à un système d'alimentation et/ou de drainage de fluide.

2. Système selon la revendication 1, dans lequel le cathéter comprend une pointe de guidage à ou près de l'extrémité distale de la tige longitudinale, ou la tige du cathéter a une lumière de fil de guidage appropriée pour faire glisser le cathéter sur un fil de guidage.

3. Système selon l'une quelconque des revendications précédentes, dans lequel la bobine de captation du dispositif d'implantation comprend totalement ou partiellement la zone d'ablation.

4. Système selon l'une quelconque des revendications précédentes, dans lequel la bobine émettrice comprend un matériau concentrant le flux.

5. Système selon l'une quelconque des revendications précédentes, dans lequel la bobine émettrice comprend un axe central sensiblement parallèle à l'axe longitudinal du cathéter.

6. Système selon la revendication 5, dans lequel les ballons sont conçus pour positionner, lors du gonflage, la tige du cathéter au centre ou près du centre de la veine et/ou du dispositif d'implantation, de préférence par une symétrie cylindrique substantielle des zones extérieures les plus externes des ballons gonflés par rapport à l'axe longitudinal du cathéter.

7. Système selon l'une quelconque des revendications 1 à 6, dans lequel au moins l'un des ballons est conçu pour isoler thermiquement la zone d'ablation du sang dans la veine lors du gonflage du ballon.

8. Système selon l'une quelconque des revendications précédentes 1 à 7, dans lequel le dispositif d'implantation comprend un axe central, et dans lequel le dispositif d'implantation est expansible radialement dans des directions perpendiculaires à son axe central, et dans lequel au moins l'un des ballons est dégonflé et destiné à supporter le dispositif d'implantation non expansé, et dans lequel au moins l'un des ballons destinés à supporter le dispositif d'implantation est capable de résister à des pressions suffisantes pour dilater radialement le dispositif d'implantation.

9. Système selon la revendication 8, dans lequel le cathéter comprend une gaine de protection mobile autour de la tige du cathéter, appropriée pour la protection du dispositif d'implant non expansé supporté par un ballonnet non gonflé.

10. Système selon l'une quelconque des revendications 1 à 9, dans lequel au moins l'un des ballons est un ballon de perfusion, ou la tige du cathéter comprend un canal de perfusion.

11. Système selon l'une quelconque des revendications 1 à 10, dans lequel au moins un des ballons est revêtu d'un anticoagulant, de préférence du sulfate d'héparine.

12. Système selon l'une quelconque des revendications précédentes, dans lequel le dispositif d'implantation comprend un revêtement thermoactif avec une température d'activation supérieure à une température prédéfinie, de préférence supérieure à 37°C, plus préférablement supérieure à 40°C, telle que 41°C, 42°C, 43°C, 44°C, 45°C, pour activer le revêtement thermoactif simultanément à et/ou après le chauffage de la zone d'ablation, dans lequel de préférence le dispositif d'implantation comprend des substances qui sont libérées du dispositif d'implantation lors de ou après l'activation du revêtement thermoactif.

13. Système selon l'une quelconque des revendications précédentes, dans lequel le dispositif d'implantation présente un axe central (L), dans lequel le dispositif d'implantation est extensible radialement dans des directions perpendiculaires à l'axe central, dans lequel la bobine réceptrice présente la zone d'ablation (412), dans lequel la zone d'ablation est extensible radialement, et dans lequel le dispositif d'implantation présente un deuxième anneau extensible radialement d'éléments en forme de diamant (410) qui sont reliés à la zone d'ablation par des entretoises (411).
